# EUROPEAN PATENT APPLICATION

(11) **EP 1 563 841 A1**
(43) Date of publication of application: **17.08.2005**
(21) Application number: 03753964.0
(22) Date of filing: 29.09.2003
(51) Int. Cl.: A61K 35/78, A61K 31/353, A61P 19/08, A61P 19/10, A61P 43/00, A23K 1/16, A23L 1/30, G01N 33/50, G01N 33/15

(54) **REMEDIES**

(30) Priority: 01.10.2002 JP 2002289056; 05.12.2002 JP 2002354414
(71) Applicant: TAKARA BIO INC., Otsu-shi, Shiga 520-2193 (JP)
(72) Inventor: OHNOGI, Hiromu, Kusatsu-shi, Shiga 525-0025 (JP); SUGIYAMA, Katsumi, Otsu-shi, Shiga 520-2134 (JP); MURAKI, Nobuko, Koka-shi, Shiga 529-1851 (JP); SAGAWA, Hiroaki, Kusatsu-shi, Shiga 525-0025 (JP); KATO, Ikunoshin, Shigaraki-cho, Koka-shi, Shiga 529-1851 (JP)
(74) Representative: VOSSIUS & PARTNER
(86) International application number: PCT/JP2003/012382
(87) International publication number: WO 2004/030683

(57) **Abstract**

The present invention relates to a therapeutic agent or prophylactic agent for a disease requiring promotion of osteogenesis or enhancement of bone morphogenetic protein production, an agent for promotion of osteogenesis or an agent for enhancement of bone morphogenetic protein production, and a food, beverage or feed for promotion of osteogenesis or enhancement of bone morphogenetic protein production, **characterized in that** each comprises as an effective ingredient a processed product derived from a plant. Also, the present invention relates to a method for measuring an enhancing action for bone morphogenetic protein production, a method for screening a substance having an enhancing action for bone morphogenetic protein production, and a method for preparing a substance having an enhancing action for bone morphogenetic protein production, each method using a specified cell.

## Description

### TECHNICAL FIELD

The present invention relates to a medicament, food, beverage or feed, which is useful for the treatment or prevention of a disease requiring bone promotion of osteogenesis or enhancement of bone morphogenetic protein production, for instance, osteoporosis, bone fracture or the like.

### BACKGROUND ART

In osseous tissues, osteogenesis and bone resorption are always repeated with maintaining a constant balance therebetween, thereby regulating bone strength and calcium concentration in blood. In the osteogenesis, osteoblasts play a key role, and in the bone resorption, osteoclasts play a key role. And it is considered that the osteoporosis is caused when the balance between the osteogenesis and the bone resorption is lost for some sort of reasons. The osteoporosis is roughly classified into postmenopausal osteoporosis caused by the lowering of estrogen secretion, and senile osteoporosis caused by aging. Besides these, secondary osteoporosis caused by an incretion or metabolism disease such as diabetes or hyperthyrea, administration of a drug such as a steroid, a disease in the digestive organ or the liver, vitamin C deficiency, immobility, oophorectomy, articular rheumatism, or the like has been known.

At present, as a therapeutic agent for osteoporosis, a drug for suppressing quantitative loss of the bone by mainly inhibiting bone resorption, such as an estrogen agent, calcitonin, or a bisphosphonate has been used. However, there are some drawbacks in the treatment with the estrogen agent, there is a strong adverse action such as an increase risk in breast cancer, uterine cancer, or a cardiac disease, in the treatment with calcitonin, the resistance to the drug is easily generated, thereby making it impossible to orally administer the drug, and in the treatment with the bisphosphonate, the absorption ratio is worsened, so that residuality is so high that excess inactivation of bone metabolism is undesirably caused. Also, a vitamin D₃ preparation has been used for the purpose of activating bone metabolism. However, its adverse action such as hypercalcemia is large while its therapeutic effect is smaller than other drugs. These conventional therapeutic agents for osteoporosis cannot recover the already progressed bone defects to the original state, so that the agents can be hardly said to be satisfactory as true therapeutic agents for osteoporosis.

Cells closely relating to the osteogenesis are osteoblasts. The osteoblasts are originated from mesenchymal stem cells which are common with cartilage cells, muscle cells, fat cells, tendon cells or the like. In the process of differentiation, the osteoblasts become mature osteoblasts through the pre-osteoblasts. The osteoblasts produce in a large amount extracellular matrices, such as collagen, a constituent of the bone in the maturation process, and also express an alkaline phosphatase to cause sedimentation of calcium phosphate to the matrices. A part of the osteoblasts is thus embedded in the calcified matrix, and further differentiated into osteocytes.

The amount of the bone in human shows its maximum at 20 to 30 years of age, and decreases thereafter. With aging, the number of osteoblasts in the osseous tissues decreases, and the differentiation ability from mesenchymal stem cells to osteoblasts is lowered. On the other hand, the number of fat cells derived from the mesenchymal stem cells similar to the osteoblasts is said to increase with aging. Therefore, it is considered to be effective from the viewpoint of prevention or treatment of osteoporosis that the osteogenesis is enhanced by increasing the amount of the osteoblasts in the growth stage of the bone and the osteocytes, and more selectively promoting the differentiation from the mesenchymal stem cells to osteoblasts in the senile stage or post menopausal stage.

Recently, developments have been made on a drug for promoting osteogenesis, and there have been disclosed the action for bone morphogenetic promotion by benzopyran derivatives (for instance, see JP-A-Hei-7-291983), phenyl-substituted hydroxycyclopentenone analogues (for instance, see JP-A-Hei-11-43460), prostaglandin A1 analogues (for instance, see JP-A-Hei-11-43461), benzothiepin derivatives (for instance, see JP-2000-109480 A), chromone derivatives (for instance, see JP-2001-139571 A). However, the evaluations for efficacy and safety are not yet satisfactory, so that these drugs have not reached a practical stage.

There has been found that a protein factor for inducing osteogenesis in the bone matrix, and named bone morphogenetic protein (hereinafter referred to as "BMP" in some cases). The entity thereof has been unknown for a long period of time. Taking the opportunity of cloning four kinds of BMP genes, it has been presently known that a dozen or so kinds of molecular species exist widely in animals beyond species. The BMP acts on pre-osteoblasts to increase alkaline phosphatase activity, responsiveness to parathyroid hormone, osteocalcine production, collagen synthesis or the like, thereby inducing the differentiation into osteoblasts. The BMP assigns the differentiation into cartilage cells, osteoblasts, or fat cells, depending upon the differentiation stage of the undifferentiated mesenchymal stem cells. The BMP suppresses the differentiation into muscles of the myoblasts, and converts to the differentiation into osteoblasts. In addition, it has been known that the BMP has an inducing activity for a secondary growth factor such as insulin-like growth factor from osteoblasts. The osteogenesis is induced by administering the BMP together with a carrier subcutaneously or intramuscularly. Among the recombinant human BMPs, BMP-2, -4, -5, -6, and -7 are found to have activities for inducing osteogenesis alone. Especially, there has been known that recombinant human BMP-2 possesses a potent osteogenetic activity, and recovers defective tissues in bone defect model of a rat, sheep, a dog, a monkey and the like. In addition, there have been reported that BMP-4 and -5 are involved in the healing process of bone fracture, and that BMP-6 is involved in endochondral ossification. Also, there have been known that BMP-12 has an activity for forming ligaments and tendons, and that BMP-13 has an activity for forming cartilages. Further, there has been pointed out that the BMP is associated with senile osteoporosis because the lowering in the amount of the BMP in the bone matrix and the lowering in sensitivity of the osteoblasts to the BMP are found in aged animals or elderly human individuals.

The BMP plays an important role not only in osteogenesis but also in the developmental process. BMP-2, -4, -7, -8, -11 and the like are involved in formation of dorsabdominal axis or mesodermal formation, cardiogenesis, nephrogenesis, oculogenesis, spermatogenesis and the like. A knockout animal of BMP shows a lethal or severe disorder. As described above, the BMP is essential for a living body, and has been known to possess a variety of physiological activities.

Since the BMP shows various kinds of actions mentioned above, the BMP itself has been tried to be directly utilized as a protein preparation in the treatment of osteoporosis, bone fracture or the like. However, the BMP is a protein, there arise problems in the limitations of the administration methods and occurrence of resistance. In addition, since the receptors for the BMP are widely expressed in numerous tissues, when the preparation is administered to a whole body, there is a risk that tissues other than the bones may be affected. Because of these drawbacks, the actual use of the BMP *per se* as a therapeutic agent is accompanied by various limitations. However, if the BMP production can be arbitrarily enhanced in a desired tissue site, not by an external administration, it is considered that the BMP is effective for treatment or prevention of a disease requiring enhancement of BMP production, such as osteoporosis or bone fracture. In recent years, there has been disclosed that a specified compound having two aromatic systems (see, for instance, WO 97/15308), or a statin compound such as mevastatin, lovastatin, pravastatin or simvastatin (see, for instance, WO 98/25460) possesses an enhancing activity for BMP-2 production. Also, there has been disclosed that helioxantin (see, for instance, JP-A-Hei-8-245386) or a condensed thiophene derivative (see, for instance, JP-A-Hei-9-151132) has an activity for enhancing the action of the BMP. However, these are still unsatisfactory in the evaluation of safety and efficacy, and have not reached the stage of actual use.

In recent years, development has been made to utilize the BMP in osteoanagenetic medicine, and there has been tried to obtain a therapeutic effect by implanting a complex of the BMP and the carrier with an implanting material to a bone fracture site. However, since the BMP is brought into a living body in a large amount, there arise some problems from the aspects of safety and economic advantages. It is considered that the drawback can be avoided by enhancing the BMP production or promoting the osteogenesis in place of administration of BMP, but a satisfactory means for actual use has not yet been known.

As described above, it is considered that by promoting the osteogenesis or enhancing the BMP production, various diseases associated therewith can be treated or prevented. However, substances, means and the like capable of appropriately promoting the osteogenesis or enhancing the BMP production as desired without showing toxicity or adverse actions have not yet been known.

*Angelica keiskei* koidz. is a large-scaled perennial plant belonging to Umbelliferae, and a variety of health-promoting effects therefor have been known. For instance, as physiological actions owned by *Angelica keiskei* koidz., prophylactic effect for hypertension, antibacterial action, anti-tumor action, suppressive action for gastric acid secretion, anti-cancerous effect, enhancing effect for nerve growth factor production, enhancing action for hepatocyte growth factor production and the like have been known (see, for instance, WO 01/76614). However, its promoting action for osteogenesis or enhancing action for BMP production has not been known.

*Apium* is a plant belonging to Umbelliferae, and a variety of physiological actions therefor have been known. As the physiological actions of *Apium,* anti-blood coagulating action, carcinostatic action and the like have been known. However, its promoting action for osteogenesis or enhancing action for BMP production has not been known.

*Lilium* is a plant belonging to Liliaceae, and a variety of physiological actions therefor have been known. As the physiological actions of *Lilium,* resolution of inflammation, diuretic action, cough-calming action, sedative action and the like have been known. However, the promoting action for osteogenesis or enhancing action for BMP production has not been known.

Aloe is a plant belonging to Liliaceae, and a variety of physiological actions therefor have been known. As the physiological actions of aloe, anti-tumor action, mitogenic activity, immunopotentiating activity, healing action for frostbite, antibacterial action, anti-allergic action, anti-inflammatory action, healing action for a burn, action for lowering blood sugar level, moisturizing action and the like have been known. However, its promoting action for osteogenesis or enhancing action for BMP production has not been known.

*Artemisia L.* is a plant belonging to Compositae, and a variety of physiological actions therefor have been known. As the physiological actions of *Artemisia L.,* antibacterial action, sedative action, digestive action for active oxygen, hemostatic action, anti-histamine action and the like have been known. However, its promoting action for osteogenesis or enhancing action for BMP production has not been known.

### DISCLOSURE OF INVENTION

An object of the present invention is to develop a composition having promoting action for osteogenesis or enhancing action for bone morphogenetic protein production suitable as food materials and medicament materials, which is derived from a natural product and safe, and is capable of being conveniently taken, and to provide a medicament, food, beverage or feed using the composition.

Summarizing the present invention, first to third inventions of the present invention relate to a therapeutic agent or prophylactic agent for a disease requiring promotion of osteogenesis or enhancement of bone morphogenetic protein production; an agent for promotion of osteogenesis or an agent for enhancement of BMP production; and a food, beverage or feed for promotion of osteogenesis or enhancement of BMP production, characterized in that each comprises as an effective ingredient a processed product derived from a plant selected from the following (a) to (c):
(a) a processed product derived from a plant belonging to Umbelliferae;
(b) a processed product derived from a plant belonging to Liliaceae; and
(c) a processed product derived from a plant belonging to Compositae.

In the first to third inventions of the present invention, as the plant, *Angelica keiskei* koidz., *Apium, Lilium,* aloe or *Artemisia L.* is suitably used.

Fourth to sixth inventions of the present invention relate to a therapeutic agent or prophylactic agent for a disease requiring promotion of osteogenesis or enhancement of BMP production; an agent for promotion of osteogenesis or an agent for enhancement of BMP production; and a food, beverage or feed for promotion of osteogenesis or enhancement of BMP production, characterized in that each comprises as an effective ingredient a compound represented by the following formula (A): 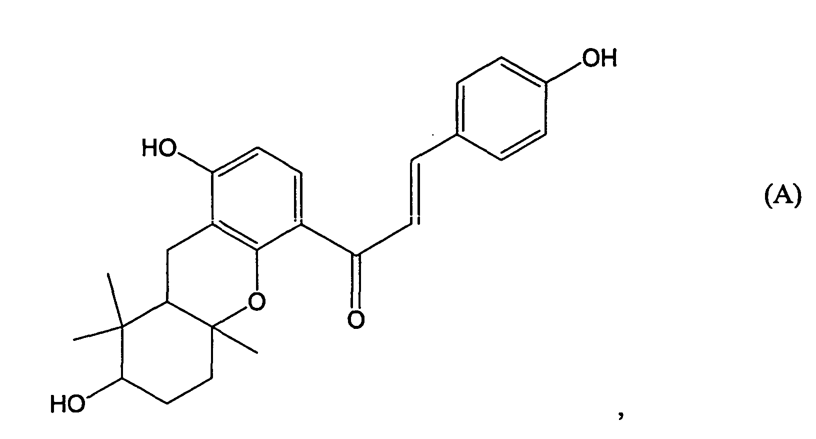 a derivative thereof or a salt thereof.

The seventh invention of the present invention relates to a method for measuring an enhancing action for BMP production, characterized in that the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a) as an index for an enhancing action for BMP production of the test substance.

The eighth invention of the present invention relates to a method for screening a substance having an enhancing action for BMP production, characterized in that the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a) wherein the test substance is determined to have an enhancing action for BMP production when the amount of BMP is larger than that of a case where the cells are cultured without contact of the test substance or with contact of a control substance having an enhancing action for BMP production.

The ninth invention of the present invention relates to a method for preparing a substance having a n enhancing action for BMP production, characterized in that the method comprises the following steps of:
(a) obtaining a substance having an enhancing action for BMP production; and
(b) measuring the enhancing action for BMP production of the substance obtained in the step (a) using the measurement method as defined in the seventh invention of the present invention.

In addition, the present invention relates to a method for preparing a substance having an enhancing action for BMP production, characterized in that the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a), wherein the test substance is determined to have an enhancing action for BMP production when the amount of BMP is larger than that of a case where the cells are cultured without contact of the test substance or with contact of a control substance having an enhancing action for BMP production, thereby giving the test substance as a substance having an enhancing action for BMP production.

### BRIEF DESCRIPTION OF THE DRAWINGS

Figure 1 is a graph showing mass spectrum of the compound **a**.
Figure 2 is a graph showing ¹H-NMR spectrum of the compound **a**.
Figure 3 is a graph showing ¹³C-NMR spectrum of the compound a.

### BEST MODE FOR CARRYING OUT THE INVENTION

The terms "enhancing action for BMP production" and "enhancing activity for BMP production" as used herein each refers to provide enhancement of BMP production and a function for enhancing BMP production, and is not intended to particularly strictly distinguish in its meaning. In addition, the term "enhance" encompasses an embodiment in which the amount of the desired substance is increased after the action of the effective ingredient of the present invention as compared to that before the action, as well as an embodiment (induction) in which the desired substance is produced by the action of the effective ingredient of the present invention. In addition, any of the substances listed as the effective ingredient in the present specification can be used alone or in admixture of two or more kinds in the present invention.

As the effective ingredient, a processed product derived from a plant selected from a processed product derived from a plant belonging to Umbelliferae; a processed product derived from a plant belonging to Liliaceae; and a processed product derived from a plant belonging to Compositae can be used.

In the present invention, the plant belonging to Umbelliferae is a plant belonging to Umbelliferae of Angiospermopsida, and exemplified by, for instance, *Angelica keiskei* koidz., *Apium, Oenanthe javanica, Cryptotaenia japonica Hassk, Angelica pubescens, Daucus,* and the like. In the present invention, *Angelica keiskei* koidz. and *Apium,* which are large-scaled perennial plants belonging to Umbelliferae, can be especially suitably used.

In the present invention, the plant belonging to Liliaceae is exemplified by aloe, *Lilium, Tricyrtis hirta, Chionographis japonica, Japonolirion saitoi, Heloniopsis orientalis, Hemerocallis, Hosta, Allium fistulosum, Allium sativum Linn, Allium tuberosum, Fritillaria camtschatcensis, Tulipa gesneriana, Erythronium japonicum, Tulipa edulis, Polygonatum odoratum var. pluriflorum, Streptopus var. japonicus, Clintonia udensis, Smilacina japonica, Maianthemum dilatatum, Disporum smilacium, Trillium smallii, Paris tetraphylla, Rohdea japonica, Ophiopogon japonicus, Liriope muscari, Aletris spicata, Colchicum autumnale L., Scolla scilloides, Narcissus, Clivia nobilis, Lycoris radiata var. radiata, Crinum asiaticum Linn. var. japonicum Baker,* and the like. In the present invention, aloe and *Lilium* are especially preferably used.

Here, aloe refers to those classified in the genus *Aloe L.,* and for instance, any of *Aloe ferox Miller, Aloe arborescen Miller var. natalensis Berger, Aloe barbadenisis Miller* and the like can be used. Also, *Aloe arborescen Miller var. natalensis Berger* may be referred to as *Aloe arborescens* according to plant taxonomy, which is also encompassed in aloe in the present specification. In addition, *Lilium* refers to those classified in the genus *Lilium,* and, for instance, any of *Lilium longiflorum, Lilium speciosum Thunb, Lilium maculatum, Lilium auratum, Lilium lancifolium* and the like can be used.

In the present invention, the plant belonging to Compositae is exemplified by *Artemisia indica var. maximowiczii,* KWANGHWA MUGWWORT, *Cirsium yozoense, Arctium lappa L., Chrysanthemum coronarium, Erigeron philadelphicus Linn, Aster tataricus, Kalimeris yomena Kitam, Eupatorium fortunei, Ambrosia artemisiaefolia var. elatior, Xanthium strumarium, Dahlia pinnata, Cosmos bipinnatus Cosmos, Helianthus, Gnaphalium affine,* *Leontopodium japonicum, Gerbera hybrid, Petasites japonicus, Arnica unalaschkensis var. tschonoskyi, Senecio nemorensis, Veronica, Ixeris dentata, Lactuca indica, Taraxacum, Crepis hokkaidoensis* and the like. In the present invention, *Artemisia indica var. maximowiczii* and KWANGHWA MUGWWORT are especially preferably used.

In addition, the plant usable in the present invention is not particularly limited, and fruit, seed, seed coat, flower, leaf, stem, root, root stem and/or whole plant can be directly used.

The processed product derived from the plant usable in the present invention is not particularly limited, as long as the processed product has a promoting action for osteogenesis or enhancing action for BMP production. The processed product refers to, for instance, an extract, a powder, a squeezed juice, a pulverized product, a chemically processed product, or an enzymatically processed product, and is especially preferably exemplified by an extract, a powder and a squeezed juice. The form of the processed product derived from the plant is not particularly limited as long as the product can be used as the effective ingredient of the present invention.

In the present invention, the extract refers to a substance obtained through the process of carrying out the extraction procedure with an extraction solvent. The extraction can be carried out as follows by a known extraction method. For instance, the raw material is powdered or cut into thin pieces, and thereafter extracted in a batch process or continuous process using a solvent. The extraction solvent used upon obtaining an extract is not particularly limited, and includes, for instance, hydrophilic or lipophilic solvents such as water, hexane, chloroform, alcohols such as ethanol, methanol and isopropyl alcohol, ketones such as acetone and methyl ethyl ketone, methyl acetate, and ethyl acetate, which can be used alone or properly as a mixed solution as desired. The amount of the extraction solvent may be appropriately determined. Usually, the extraction solvent may be used in an amount of preferably from 0.1- to 100-folds that by weight of the raw materials subjected to the extraction procedure. The extraction temperature may be also appropriately determined according to its purposes. In the case of the water extraction, usually, the extraction temperature is in the range of preferably from 4° to 130°C, more preferably from 25° to 100°C. Alternatively, in the case where ethanol is contained in the solvent, the extraction temperature is suitably within the range of from 4° to 60°C. The extraction time may be also determined in consideration of extraction efficiency. It is usually preferable that the raw materials, the extraction solvent and the extraction temperature are set so that the extraction time is within the range of preferably from several seconds to several days, more preferably 5 minutes to 24 hours. The pressure during the extraction is not particularly limited, and can be properly determined as desired. The extraction procedure can be carried out by selecting the conditions as desired, for instance, under normal pressure, under pressure or reduced pressure by suction filtration or the like. The extraction procedure may be carried out, for instance, while stirring or allowing the mixture to stand. Also, the extraction procedures may be repeated several times as desired. By the above procedures, an extract derived from the plant usable in the present invention (hereinafter which may be referred to as the extract of the present invention in some cases) is obtained. The extract is subjected to such a process as filtration, centrifugation, concentration, ultrafiltration or molecular sieving as desired, whereby an extract in which the desired component having a promoting action for osteogenesis or enhancing action for BMP production (hereinafter referred to as component for promoting osteogenesis or component for enhancing BMP production, respectively) is concentrated can be prepared. The promoting action for osteogenesis or enhancing action for BMP production of the extract or concentrated extract can be conveniently determined in accordance with the method described in Example 2 or 4 set forth below. Alternatively, the plant usable in the present invention may be processed in the form of tea-leaves by a known method, and an extract obtained from the tea-leaves can be used as the extract of the present invention as long as the extract has a promoting action for osteogenesis or enhancing action for BMP production. In the present invention, two or more kinds of extracts obtained by different extraction methods can be contained to be used together.

In addition, in the present invention, a fraction obtained by fractionating an extract derived from the plant usable in the present invention by a known method, or a fraction obtained by repeating the fractionation procedures a plural times is also encompassed in the extract of the present invention. The above-mentioned fractionation means include extraction, separation by precipitation, column chromatography, thin-layer chromatography, and the like. The component for promoting osteogenesis or component for enhancing BMP production can also be isolated by further proceeding the purification of the resulting fraction using the promoting action for osteogenesis or enhancing action for BMP production as an index. These components are also encompassed in the effective ingredient of the present invention.

Alternatively, the processed product derived from the plant other than the extract derived from the plant includes a powder derived from the plant. As a method for preparing a powder, for instance, a plant is dried and powdered, whereby a powder derived from a plant can be obtained.

In addition, the method for preparing a squeezed juice derived from the plant includes a known method of squeezing a plant. For instance, the method includes, but not particularly limited to, a method using a squeezer of a screw-type, a gear-type, a cutter-type or the like, or a juicer. Also, the raw materials may be cut into thin pieces or mashed as a pre-processing, and thereafter squeezed with the above-mentioned juicer or cloth or the like, whereby a squeezed juice can be obtained.

The pulverized product refers to one prepared by pulverizing the plant usable as the raw materials for the effective ingredient, and its tissue piece is generally larger than the powder. For instance, the pulverized product can be prepared by using a pulverizer. Also, the chemically processed product is not particularly limited, and refers to a product obtained by subjecting the plant to an acid processing, an alkali processing, an oxidation processing or a reducing processing. The chemically processed product can be prepared, for instance, by immersing the plant in an aqueous solution containing an inorganic acid or organic acid, such as hydrochloric acid, sulfuric acid, nitric acid, citric acid or acetic acid, or an inorganic base or organic base, such as sodium hydroxide, potassium hydroxide or ammonia. The chemically processed product includes all those derived from plants subjected to chemical processing as mentioned above. The enzymatically processed product refers, for instance, to an enzymatically processed product with pectinase, cellulase, xylanase, amylase, mannanase, or glucosidase, an enzymatic reaction product by a microorganism (for instance, a fermented product) or the like. The enzymatically processed product can be prepared, for instance, by allowing the above-mentioned enzyme to act on the plant in an appropriate buffer. The enzymatically processed product includes all those derived from plants subjected to the enzymatic processing as mentioned above. Further, the processed product derived from the plant of the present invention encompasses, for instance, juice obtained by cutting stem of the above-mentioned plant and obtaining juice from its cross section.

In the present invention, the shape of the processed product derived from the plant is not particularly limited as long as the processed product has a promoting action for osteogenesis or enhancing action for BMP production, and the processed product may take any form of powder, solid or liquid. In addition, The processed product can be used as a processed product derived from the plant of the present invention in the form of a granular solid prepared by, for instance, granulating the processed product by a known process. The granulation process is not particularly limited, and is exemplified by tumbling granulation, agitation granulation, fluidizing bed granulation, airflow granulation, extruding granulation, compression molding granulation, disintegration granulation, spray granulation, spray-drying granulation or the like. In addition, the processed product can be used as the processed product derived from the plant of the present invention in the form of a liquid prepared by, for instance, dissolving a powdery processed product derived from the plant in a liquid, for instance, water, an alcohol or the like.

It is especially preferable that the processed product derived from the plant in the present invention comprises the component for promoting osteogenesis or the component for enhancing BMP production in a high concentration and/or at high purity, as compared to that of the raw material plant itself. Here, the term "high concentration" means that the weight of the component for promoting osteogenesis or the component for enhancing BMP production per unit weight of the processed product derived from the plant is greater than the weight of the component for promoting osteogenesis or the component for enhancing BMP production per unit weight of the raw material plant. In addition, the term "high purity" means that the content ratio of the component for promoting osteogenesis or the component for enhancing BMP production in the processed product derived from the plant is higher than that of the raw material plant.

In addition, the present invention provides a food, beverage or feed, comprising a processed product derived from the plant in a high concentration and/or at a high purity. This means that there is provided a food, beverage or feed in which the component for promoting osteogenesis or the component for enhancing BMP production is contained in the food, beverage or feed of the present invention in a high concentration and/or at a high purity, as compared to that of a conventional food, beverage or feed.

In addition, in the present invention, the compound represented by the above-mentioned formula (A) obtainable from a processed product derived from the plant belonging to Umbelliferae, a derivative thereof or a salt thereof (which may be hereinafter referred to as the compound of the present invention) can be used as an effective ingredient for the therapeutic agent, the prophylactic agent, the agent for promotion of osteogenesis, the agent for enhancement of BMP production, the food, the beverage, or the feed of the present invention. The compound of the present invention can be each used alone or in admixture of two or more kinds. The compound may be one which is purified from a plant belonging to Umbelliferae, or one which is chemically semi-synthesized or synthesized.

A concrete method for preparing the compound represented by the formula (A) is exemplified by the method described in Example 1 set forth below. Also, for instance, a semi-synthesized product is obtained by subjecting a chalcone compound derived from a natural product as a raw material to organic synthesis, and a synthetic product is obtained by entirely carrying out organic synthesis. The method of organic synthesis may be, for instance, referred to Alessandra Lattanzi et al., *Synlett.* 2002, No. 6, p942-946; L. Claisen A. et al., *Ber. 1881,* No. 14, p2460; and the like.

The derivative of the compound represented by the above-mentioned formula (A) as used herein refers to a compound prepared by using the compound as an original compound, wherein the compound has a similar action to the compound represented by the formula (A), i.e., a promoting action for osteogenesis or enhancing action for BMP production. The derivative includes, for instance, a compound capable of being easily hydrolyzed in a body to exhibit the desired effects (prodrug), such as an ester form, an ether form, or a glycoside form of the compound represented by the above-mentioned formula (A). The prodrug may be prepared in accordance with a known method. The derivative may be a salt thereof.

In addition, in the compound of the present invention, as the salt, a pharmacologically acceptable salt is preferable. The salt usable in the present invention is exemplified by alkali metal salts, alkaline earth metal salts, salts with an organic base and the like. As the salt, a pharmacologically acceptable salt is preferable. Here, the pharmacologically acceptable salt means a salt which is substantially nontoxic against an organism. The salts include, for instance, salts with sodium, potassium, calcium, magnesium, ammonium or protonated benzathine (N,N'-di-benzylethylenediamine), choline, ethanolamine, diethanolamine, ethylenediamine, meglamine (N-methylglucamine), benethamine (N-benzylphenetylamine), piperazine or tolomethamine (2-amino-2-hydroxymethyl-1,3-propanediol).

Incidentally, in the present invention, the processed product derived from the plant usable in the present invention and/or the compound of the present invention may be referred to as the effective ingredient of the present invention, and the therapeutic agent or prophylactic agent for a disease requiring promotion of osteogenesis or enhancement of BMP production, comprising the effective ingredient of the present invention may be referred to as the therapeutic agent or prophylactic agent of the present invention in some cases.

No toxicity is especially found in the effective ingredient according to the present invention as mentioned later. Also, there is no risk of the onset of adverse actions. For these reasons, the disease can be safely and appropriately treated and prevented. Therefore, the therapeutic agent, the prophylactic agent, the agent for promotion of osteogenesis, the agent for enhancement of BMP production, the food, the beverage or the feed of the present invention, each comprising the effective ingredient, is effective for treating or preventing a disease requiring promotion of osteogenesis or enhancement of BMP production.

The promoting action for osteogenesis in the present invention is not particularly limited, as long as a morphogenetic action of bones and cartilages is promoted. For instance, the promoting action for osteogenesis is exemplified by a promoting action for differentiation of mesenchymal stem cells into osteoblasts, a promoting action for differentiation of undifferentiated mesenchymal stem cells into osteoblasts, a promoting action for differentiation of preosteoblasts into osteoblasts, a promoting action for formation of bone matrix, an action of calcification of bone matrix, a promoting action for differentiation of osteoblasts into osteocytes, an inducing action for endochondral ossification, and the like. In addition, the presence or absence of the promoting action for osteogenesis is not particularly limited. The presence or absence can be conveniently determined, for instance, as an action for promoting differentiation from mesenchymal stem cells into osteoblasts according to the method described in Example 2 set forth below. Here, the term "promoting" encompasses "inducing."

In the present invention, BMP is exemplified by, for instance, BMP-1, BMP-2, BMP-3, BMP-4, BMP-5, BMP-6, BMP-7, BMP-8, BMP-9, BMP-10, BMP-11, BMP-12, BMP-13, BMP-14, BMP-15 and the like, and especially preferably BMP-2, BMP-4 or BMP-7 is exemplified. Also, the presence or absence of the enhancing action for BMP production is not particularly limited. The presence or absence can be conveniently determined according to the method described in Example 4 set forth below.

The BMP is a factor for potently promoting the formation of bones, cartilages, ligaments, tendons and the like, and is thought to act on preosteoblasts to differentiate into osteoblasts, to be involved in generation, growth, and remolding of the bone, and the healing process of the bone fracture. In addition, the BMP acts on undifferentiated mesenchymal stem cells to assign the differentiation into chondroblasts, osteoblasts or fat cells depending upon the differentiation stages, whereby greatly involved in the maturation and proliferation of mesenchymal stem cells. Furthermore, in the process of the development of an individual, the BMP plays a key role in formation of dorsabdominal axis, mesodermal formation or the like. Among the BMPs, BMP-2, -4, and -7 have especially potent bone morphogenetic activities, so that a recombinant human BMP-2 can act on a bone-defective animal to restore the bone defects. The details on the recombinant human BMP-2 are to be referred to, for instance, J. M. Wozney et al., Science **242** 1528-1534 (1988). Also, in the dental field, regeneration of periodontium such as alveolar bone cementum or periodontal ligament tissues by BMP-2 has been confirmed.

In the present invention, the disease requiring promotion of osteogenesis or enhancement of BMP production is exemplified by osteoporosis (for instance, chronic osteoporosis, osteoporosis caused by abnormality of hormonal balance in postmenopausal stage, secondary osteoporosis accompanying diabetes or an adverse action of steroid or the like), bone fracture, bone refracture, bone defects, dysosteogenesis, osteomalacia, osteonecrosis of Behget's disease, ankylosing spondylitis, chronic articular rheumatism, osteoarthritis, osteoarthritis involving cartilages, periodontal diseases, periodontium defects in periodontal diseases, tooth root or alveolar defects, ridge formation, and recovery of palatoschisis. In addition, the therapeutic agent or prophylactic agent of the present invention can be also used as an osseous tissue restoration agent after surgery for multiple myeloma, lung cancer, breast cancer, and the like. Furthermore, the therapeutic agent or prophylactic agent of the present invention can be used for the osteoanagenesis purposes in the field of regenerative medicine. Concretely, the therapeutic agent or prophylactic agent of the present invention can be used for activating or stabilizing artificial bone or artificial tooth root. Also, cells can be taken from a living body of a patient before having the disease or a patient having the disease, and allowing the therapeutic agent or prophylactic agent of the present invention to act *in vitro* to form regenerated osseous tissues, and thereafter returning the cells to the living body of the patient.

The therapeutic agent or prophylactic agent of the present invention includes one formed into a preparation by combining the above-mentioned effective ingredient according to the present invention with a known medicament. The effective ingredient of the present invention can be also used together with, for instance, a drug for inhibiting the absorption of the bone, such as estrogen, calcitonin, activated vitamin D₃, bisphosphonate or the like. Besides them, the effective ingredient of the present invention can be used together with the BMP. By the addition, as described in Example 13, synergistic effects regarding the promotion of osteogenesis can be expected. Here, as the BMP, the above-mentioned recombinant human BMP-2 is preferably used. Therefore, a preferred embodiment of the therapeutic agent or prophylactic agent of the present invention includes an agent comprising the above-mentioned processed product derived from the plant and/or the compound of the present invention, and a recombinant human BMP-2. The constitution is also a preferred embodiment in the agent for promoting osteogenesis, the agent for enhancing BMP production, the food, the beverage, and the feed of the present invention.

The therapeutic agent or prophylactic agent of the present invention is usually manufactured by formulating the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like is optionally added thereto, so that a solid agent such as a tablet, a granule, a powder, a fine powder, and a capsule, or a liquid agent such as a common liquid agent, a suspension agent or an emulsion agent can be formed. In addition, there can be also made into a dry product which can be made liquid by adding an appropriate liquid carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for instance, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a non-orally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, an osmotic regulator, a soothing agent, or the like as desired. It is also possible to produce a solid composition which is dissolved in sterile water or a sterile solvent for injection before use.

The external preparation includes solid, semi-solid or liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquid agents for transmucosal administration; ointments such as oily ointments and hydrophilic ointments; medical adhesives for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is in an amount so that the effective ingredient can be preferably administered within the dose described below in consideration of administration form, administration method and the like of the preparation. For instance, when a concentrate prepared by concentrating an extract obtained by the extraction procedure of extracting 1 g of a dry product of the plant usable as the raw material in the present invention with 20 ml of water as an extraction solvent to a volume of 5 ml is used as an effective ingredient, the content is usually from 0.001 to 100% by weight, preferably from 0.01 to 90% by weight, more preferably from 0.1 to 80% by weight, of 100% by weight of the therapeutic agent or prophylactic agent. In addition, when the compound of the present invention is used as the effective ingredient, the content of the compound is, but not particularly limited thereto, usually from 0.000001 to 100% by weight, preferably from 0.00001 to 90% by weight, more preferably from 0.0001 to 80% by weight, of 100% by weight of the therapeutic agent or prophylactic agent.

Here, when the effective ingredient of the present invention is used together with the above-mentioned recombinant BMP-2, the recombinant BMP-2 is preferably used in an amount of usually from 0.1 to 100000 parts by weight or so, based on 100 parts by weight of the effective ingredient. The ratio upon the combined use of the effective ingredient and the recombinant BMP-2 is the same for the agent for promoting osteogenesis, the agent for enhancing BMP production, the food, the beverage, and the feed described later.

The therapeutic agent or prophylactic agent of the present invention is administered via an administration route appropriate for each of the preparation form. The administration method is also not limited to specific one. The agent can be administered internally, externally (or topically) or by injection. The injection can be administered, for instance, intravenously, intramuscularly, subcutaneously, intracutaneously, or the like. As to an external preparation, for instance, a suppository may be administered according to its proper administration method.

The dose of the therapeutic agent or prophylactic agent of the present invention is changeable and properly set depending upon its preparation form, administration method, purpose of use, and age, body weight, symptom or the like of a patient to which the therapeutic agent or prophylactic agent is applied, or the like. Generally, for instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, the dose of the agent, in terms of the dose of the above-mentioned effective ingredient contained in the preparation, is from 0.0001 mg to 2000 mg/kg body weight, preferably from 0.001 mg to 1000 mg/kg body weight, more preferably from 0.01 mg to 100 mg/kg body weight, per day for human (for instance, adult). In addition, when the compound of the present invention is used as the effective ingredient, the dose of the compound is, but not particularly limited thereto, from 0.0001 µg to 2000 mg/kg body weight, preferably from 0.001 µg to 1000 mg/kg body weight, more preferably from 0.01 µg to 100 mg/kg body weight per day for human (for instance, adult). As a matter of course, the dose varies depending upon various conditions, for instance, the kinds of the extraction solvent, the amount of the solvent used and the like, so that an amount smaller than the dose mentioned above may be sufficient, or an amount exceeding the dose range may be required. Administration may be carried out once or in several divided portions in a day within the desired dose range. Also, the therapeutic agent or prophylactic agent of the present invention can be directly orally administered, or the agent can be added to any foodstuffs to be taken on a daily basis.

In addition, the present invention can provide an agent for promoting osteogenesis and an agent for enhancing BMP production, each comprising the above-mentioned effective ingredient. The agent for promoting osteogenesis and the agent for enhancing BMP production may be the above-mentioned effective ingredient itself, or a composition comprising the above-mentioned effective ingredient. The agent for promoting osteogenesis and the agent for enhancing BMP production may be prepared by, for instance, formulating the above-mentioned effective ingredient with other ingredients which can be used for the same application as the effective ingredient, and forming into a form of reagent usually used according to the above-mentioned process for preparing the therapeutic agent or prophylactic agent. The content of the above-mentioned effective ingredient in the agent for promoting osteogenesis and the agent for enhancing BMP production is not particularly limited, as long as the content is in an amount so that the desired effects of the present invention can be exhibited in consideration of administration method, purpose of use or the like of the agent for promoting osteogenesis and the agent for enhancing BMP production. For instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, the content is usually from 0.001 to 100% by weight, preferably from 0.01 to 90% by weight, more preferably from 0.1 to 80% by weight, of 100% by weight of the agent for promoting osteogenesis and the agent for enhancing BMP production. In addition, when the compound of the present invention is used as the effective ingredient, the content of the compound is, but not particularly limited thereto, usually from 0.000001 to 100% by weight, preferably from 0.00001 to 90% by weight, more preferably from 0.0001 to 80% by weight, of 100% by weight of the agent for promoting osteogenesis and the agent for enhancing BMP production.

Also, the amount of the agent for promoting osteogenesis or the agent for enhancing BMP production used is not particularly limited, as long as the desired effects of the present invention can be exhibited. Especially in the case where the agent is administered to a living body, the agent may be preferably used in an amount so that the effective ingredient can be administered within the dose range of the effective ingredient for the above-mentioned therapeutic agent or prophylactic agent. The agent for promoting osteogenesis or the agent for enhancing BMP production is useful for a disease involved in promotion of osteogenesis or enhancement of BMP production.

In addition, the agent for promoting osteogenesis or the agent for enhancing BMP production can be used by containing in the implant. Therefore, by using in the implant, for instance, in bone fracture, the osteosynthesis can be promoted, whereby the healing of the bone fracture or integration of the implant and the osseous tissues can be accelerated. The term implant as used herein means an instrument at least partially introduced into a body during a surgery, and is used for breakage or damages of joints, bones, teeth, ligaments, tendons, or the like. Also, the implant may permanently remain in the body, and is reabsorbed in an organism. Here, the agent for promoting osteogenesis or the agent for enhancing BMP production may be contained in the internal of the implant, or may be contained by coating the implant surface with the agent. The content of the agent for promoting osteogenesis or the agent for enhancing BMP production of the present invention may be properly set so that the desired effects can be obtained. The implant can be prepared by a known method using the effective ingredient of the present invention.

In addition, the agent for promoting osteogenesis or the agent for enhancing BMP production of the present invention can be used to be contained in a dentifrice. The dentifrice can regenerate periodontal tissues, prevent periodontal inflammation and promote the re-calcification of teeth. The content of the agent for promoting osteogenesis or the agent for enhancing BMP production of the present invention may be properly set so that the desired effects can be obtained. The dentifrice can be prepared by a known method using the effective ingredient of the present invention.

In addition, the agent for promoting osteogenesis and the agent for enhancing BMP production is useful for screening of drugs for diseases involving bones. Furthermore, the agent for promoting osteogenesis and the agent for enhancing BMP production is useful for functional studies relating to physical changes in the bones.

No toxicity is especially found in the effective ingredient according to the present invention as described later. Also, there is no risk of the onset of adverse actions. For these reasons, according to the effective ingredient, the promoting action for osteogenesis and the enhancing action for BMP production can be safely and appropriately exhibited. Therefore, the medicament, food, beverage or feed of the present invention comprising the effective ingredient is effective for treating or preventing a disease requiring promotion of osteogenesis or enhancement of BMP production.

In addition, the present invention provides a food, beverage or feed for promotion of osteogenesis or enhancement of BMP production, comprising the above-mentioned effective ingredient. The term "comprising(ed)" means containing(ed), adding(ed), and/or diluting(ed). The food, beverage or feed of the present invention is very useful in amelioration of symptoms and prevention of a disease requiring promotion of osteogenesis or enhancement of BMP production.

As used herein, the above-mentioned term "containing(ed)" refers to an embodiment of containing the effective ingredient usable in the present invention in the food, beverage or feed; the above-mentioned term "adding(ed)" refers to an embodiment of adding the effective ingredient usable in the present invention to a raw material for the food, beverage or feed; and the above-mentioned term "diluting(ed)" refers to an embodiment of adding a raw material for the food, beverage or feed to the effective ingredient usable in the present invention.

The process for preparing the food, beverage or feed of the present invention is not particularly limited. For instance, formulation, cooking, processing, and the like can be carried out in accordance with those generally employed for foods, beverages or feeds, and the food, beverage or feed of the present invention can be prepared by the general methods for preparing a food, beverage or feed, as long as the resulting food, beverage or feed may contain the above-mentioned effective ingredient of the present invention, wherein the effective ingredient has promoting action for osteogenesis and the enhancing action for BMP production.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao,* and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chewing gums, candies, chocolates, biscuits, sweet bun, cake, rice cake snacks and rice snacks; alcohol beverages such as *sake,* Chinese liquor, wine, whiskey, Japanese distilled liquor (*shochu*)*,* vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods (soups and the like); spices; and the like, in which each of the foods and beverages comprises the above-mentioned ingredient according to the present invention. In addition, as the food of the present invention, chewing gums, candies and the like are especially preferable. Since the food is chewed in the mouth for a given period of time, if the effective ingredient of the present invention is contained in the food, the effects exhibited by the effective ingredient of the present invention, for instance, regeneration effects of periodontal tissues or the promoting effects of re-calcification of teeth can be more effectively exhibited.

In the food or beverage of the present invention, its shape is not particularly limited, as long as the above-mentioned effective ingredient is contained, added and/or diluted, alone or in plurality, and the effective ingredient is contained in an amount necessary for exhibiting its promoting action for osteogenesis and the enhancing action for BMP production. For instance, the shape includes those which can be taken orally such as tablets, granules and capsules.

In addition, as to the beverage of the present invention, there can be prepared into healthcare drink by mixing the effective ingredient of the present with a squeezed juice of a plant other than those belonging to Umbelliferae, Liliaceae and Compositae, for instance, a vegetable, a fruit or the like, or squeezing the plant together with these plants. For instance, the healthcare drink having promoting action for osteogenesis and the enhancing action for BMP production can be prepared by diluting a squeezed juice of *Angelica keiskei* *koidz.,Apium, Lilium,* aloe or *Artemisia L.* with water, or mixing the squeezed juice with a squeezed juice *of Brassica Rapa* var. *pervidis* (*komatsuna*), Japanese turnip, Qing gin cai, tomato, mandarin orange, lemon, grapefruit, kiwi, spinach, radish, Japanese radish (*daikon*), Chinese cabbage, cabbage, sunny lettuce, lettuce, okra, green pepper, cucumber, kidney beans, green soybeans, pea, Indian corn, Rocket, loquat, *Citrus natsudaidai, amanatsu,* or the like, cow's milk, soybean milk or the like.

In addition, as an alcoholic beverage, which is one embodiment of the beverage of the present invention, there can be provided one prepared by immersing a plant belonging to Umbelliferae, a plant belonging to Liliaceae and/or a plant belonging to Compositae, for instance, *Angelica keiskei* koidz., *Apium, Lilium,* aloe and/or *Artemisia L.* in an alcohol for drinking use as it is, or as an alcoholic beverage obtainable according to a known process for preparing an alcoholic beverage for drinking.

Further, the food of the present invention encompasses a processed product derived from a plant usable as the effective ingredient in the present invention, for instance, one prepared by molding a powder or a pulverized product in the form of a tablet or the like in accordance with the known method.

The content of the above-mentioned effective ingredient in the food or beverage of the present invention is not particularly limited, and the content can be appropriately selected from the viewpoints of sensory aspect and exhibition of activity. For instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, the content of the effective ingredient is 0.1% by weight or more, preferably from 1 to 100% by weight, more preferably from 6 to 100% by weight, per 100% by weight of the food, and the content is 0.1% by weight or more, preferably from 1 to 100% by weight, more preferably from 6 to 100% by weight, per 100% by weight of the beverage. Also, when the compound of the present invention is used as the effective ingredient, the amount of the compound taken is, but not particularly limited thereto, from 0.0001% by weight or more, preferably from 0.001 to 50% by weight, more preferably from 0.006 to 10% by weight, per 100% by weight of the food, and the amount is 0.0001% by weight or more, preferably from 0.001 to 50% by weight, more preferably from 0.006 to 10% by weight, per 100% by weight of the beverage. In addition, for instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, it is preferable that the food or beverage of the present invention is taken so that the effective ingredient contained therein is preferably taken in an amount of from 0.0001 µg to 2000 g/kg body weight, preferably from 0.001 µg to 1000 mg/kg body weight, more preferably from 0.01 µg to 100 mg/kg body weight, per day for human (for instance, adult). In addition, when the compound of the present invention is used as the effective ingredient, the amount of the compound taken is, but not particularly limited thereto, from 0.0001 µg to 2000 mg/kg body weight, preferably from 0.001 µg to 1000 mg/kg body weight, more preferably from 0.01 µg to 100 mg/kg body weight, per day for human (for instance, adult). As a matter of course, the amount of taken varies depending upon various conditions, for instance, kinds of the extraction solvents, the amounts of the solvents used, and the like, so that an amount smaller than the amount of taken mentioned above may be sufficient, or an amount exceeding the range of the amount of taken may be required.

In addition, the present invention provides a feed for an organism having promoting action for osteogenesis and the enhancing action for BMP production, comprising the above-mentioned effective ingredient. In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient. The term "comprises(ing)" as used herein means contain(ing), add(ing) and/or dilute(ing) as mentioned above.

In these inventions, the organisms are, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or amelioration in physical conditioning. The organism feeding agent is exemplified by immersion agents, feed additives, and beverage additives.

According to these inventions, the same effects can be expected to be exhibited as those of the above-mentioned therapeutic agent or prophylactic agent of the present invention, on the basis of the promoting action for osteogenesis and the enhancing action for BMP production of the above-mentioned effective ingredient usable in the present invention, in the organism exemplified above for applying these. In other words, a therapeutic or prophylactic effect for a disease requiring promotion of osteogenesis or enhancement of BMP production can be exhibited in the organism.

For instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, the above-mentioned effective ingredient usable in the present invention is usually administered in an amount of preferably from 0.0001 mg to 2000 mg/kg body weight, preferably from 0.001 mg to 1000 mg/kg body weight, more preferably from 0.01 mg to 100 mg/kg body weight, per day for the subject organism. In addition, when the compound of the present invention is used as the effective ingredient, the dose of the compound is, but not particularly limited thereto, from 0.0001 µg to 2000 mg/kg body weight, preferably from 0.001 µg to 1000 mg/kg body weight, more preferably from 0.01 µg to 100 mg/kg body weight, per day for the subject organism. As a matter of course, the dose varies depending upon various conditions, for instance, kinds of the extraction solvents, the amounts of the solvents used, and the like, so that a dose smaller than the dose mentioned above may be sufficient, or a dose exceeding the range of the dose may be required. The administration can be made by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the above-mentioned effective ingredient in the feed is not particularly limited. The content can be appropriately set in accordance with its purposes. For instance, when a concentrate prepared by concentrating an extract obtained from 1 g of a dry product of the plant usable as the raw material in the present invention by the extraction procedure with 20 ml of water as an extraction solvent to a volume of 5 ml is used as an effective ingredient, the content is usually 0.1% by weight or more, preferably from 1 to 100% by weight, more preferably from 6 to 100% by weight, of 100% by weight of the feed. In addition, when the compound of the present invention is used as the effective ingredient, the content of the compound is, but not particularly limited thereto, 0.0001% by weight or more, preferably from 0.001 to 50% by weight, more preferably from 0.006 to 10% by weight, of 100% by weight of the feed.

The process for preparing the feed according to the present invention is not particularly limited, and its composition may be set in accordance with a general feed, as long as the above-mentioned effective ingredient according to the present invention having promoting action for osteogenesis and the enhancing action for BMP production may be contained in the feed prepared.

The organism feeding agent may be prepared, used and the like in the same manner as the above-mentioned feed.

The organism to which the present invention can be applied is not limited. The culturing or breeding animals include cattle such as *Equus, Bos, Porcus, Ovis, Capra, Camelus,* and *Lama;* experimental animals such as mice, rats, guinea pigs, and rabbits; poultry such as *Chrysolophus,* ducks, *Meleagris,* and *Struthioniformes;* and the pet animals includes dogs, cats, and the like, so that the feed can be widely applied.

By allowing a subject organism to take the feed comprising the above-mentioned effective ingredient usable in the present invention having promoting action for osteogenesis and the enhancing action for BMP production, or immersing a subject organism into a solution containing the above-mentioned effective ingredient usable in the present invention having promoting action for osteogenesis and the enhancing action for BMP production, the physical conditions of the cattle, experimental animals, poultry, pet animals or the like can be well sustained or ameliorated. These embodiments are one embodiment of the method of feeding an organism provided by the present invention.

Subsequently, the method for measuring an enhancing action for BMP production, the method for screening a substance having an enhancing action for BMP production and the method for preparing the substance of the present invention will be explained. All these methods are suitably used for screening of a plant to be used as a raw material in the present invention, obtainment of the effective ingredient of the present invention, evaluation for enhancing action for BMP production owned by the effective ingredient of the present invention, and owned by a composition such as a therapeutic agent or prophylactic agent, comprising the effective ingredient.

As another embodiment of the present invention, there is provided a method for measuring an enhancing action for BMP production, characterized in that the method comprises the steps of (a) culturing hybridoma obtained by using HuO9 cells or a substrain thereof, or any one of cell strains therefrom with contact of a test substance (these cells may be hereinafter collectively referred to as "the cell strains of the present invention"); and (b) measuring an amount of BMP in a culture medium obtained in the step (a) as an index for an enhancing action for BMP production of the test substance (which may be referred to as "the measurement method of the present invention").

The measurement method of the present invention clarifies that the amount of BMP production in the cells can be stably measured for the first time by using the cell strains of the present invention among various cells for BMP production, and is perfected thereby. Here, the same can be said for the method for screening a substance having an enhancing action for BMP production and the method for preparing the substance of the present invention described later.

The above-mentioned HuO9 cells (Human osteosarcomas) are marketed, and are available. In addition, the substrain of HuO9 cells is exemplified by mutants such as spontaneous mutants and artificial mutants. The above-mentioned artificial mutant can be prepared by a known mutation process, for instance, a mutation inducing agent or ultraviolet irradiation. Further, among them, the hybridoma obtained by using any one kind of cell strain can be prepared by known cell fusion procedures, and is exemplified by, for instance, cell strains obtained by a method of cell fusion with myeloma cells, and the like.

In the measurement method of the present invention, the medium usable in the culture of the cell strain usable in the present invention is not particularly limited, as long as a medium capable of growing the cells is selected, and a known marketed medium may be used. The culture time of the cell strain usable in the present invention is not particularly limited, as long as the culture time is the time required for elevating the amount of BMP production of the cells or longer. For instance, the culture time includes from several minutes to 10 days, preferably from 1 hour to 5 days, more preferably from 12 hours to 3 days. The culture temperature is not particularly limited, and the culture can be carried out at a temperature appropriate for the growth of the cell strain usable in the present invention, for instance, a temperature of from 0° to 100°C, preferably from 10° to 60°C, more preferably from 20° to 50°C.

The culture of the cell strain of the present invention with contact of the test substance in the step (a) can be carried out, for instance, by adding and mixing a test substance to a culture medium containing the cell strain at the beginning of the culture of the cell strain or during the culture, and beginning or continuing the culture. The amount of the test substance is not particularly limited, and may be properly set so that the desired effects are obtained. It is preferable that the amount of the cell strain of the present invention in the culture medium at the addition of the test substance is from 1 × 10³ to 1 × 10⁶ cells/ml or so.

After the culture for a given period of time, in the step (b), the amount of BMP produced from the cell strain of the present invention is measured. The measurement of the amount of BMP in the culture medium is not particularly limited, and can be carried out by, for instance, enzyme immunoassay method, radioimmunoassay method, Western blotting method, or biological actions of BMP, for instance, a bioassay using the ability of inducing the differentiation into osteoblasts as an index (see, for instance, Katagiri et al., BBRC **172**, No.1, p295-299 (1990); Yamaguchi et al., BBRC **120**, No.2, p366-371 (1996); Takuwa et al., BBRC **174**, No.1, p96-101 (1991)).

In the measurement method of the present invention, the enhancing action for BMP production is measured using the amount of BMP produced from the cell strain of the present invention as an index. For instance, as described in Example 4 described later, the enhancing action for BMP production of a test substance can be quantitatively measured (evaluated) by relatively expressing the amount of BMP production with contact of a test substance, wherein the amount of the BMP produced from the cell strain of the present invention without contact of a test substance is defined as 100%. In other words, when the amount of BMP exceeds 100%, the test substance can be determined to have an enhancing action for BMP production. Also, since the amount of BMP is expressed in a numerical value as a relative amount, the action can be quantitatively evaluated.

As a still another embodiment of the present invention, there is provided a method for screening a substance having an enhancing action for BMP production, characterized in that the method comprises the steps of (a) culturing the cell strain of the present invention with contact of a test substance; and (b) measuring an amount of BMP in a culture medium obtained in the step (a) wherein the test substance is determined to have an enhancing action for BMP production when the amount of BMP is larger than that of a case where the cells are cultured without contact of the test substance or with contact of a control substance having an enhancing action for BMP production (which may be hereinafter referred to as "the screening method of the present invention").

The screening method of the present invention can be carried out in the same manner as the measurement method of the present invention.

For instance, as mentioned above, the enhancing action for BMP production of a test substance can be quantitatively shown by relatively expressing the amount of BMP production with contact of a test substance, wherein the amount of the BMP produced from the cell strain of the present invention without contact of a test substance is defined as 100%. In other words, when the amount of BMP exceeds 100%, the test substance can be determined to have an enhancing action for BMP production. Also, a substance having a more excellent enhancing action for BMP production can be screened by measuring the enhancing action for BMP production for each of a test substance and a control substance having a given enhancing action for BMP production (for instance, a compound represented by the above-mentioned formula (A)), quantitatively expressing the amount of BMP in the test substance in accordance with the above-mentioned case, wherein the amount of BMP of the control substance is defined as 100%, and comparing the test substance and the control. Here, in the measurement of the enhancing action for BMP production, the above-mentioned measurement method of the present invention can be used.

As a still another embodiment of the present invention, there is provided a method for preparing a substance having an enhancing action for BMP production, characterized in that the method comprises the steps of (a) obtaining a substance having an enhancing action for BMP production; and (b) measuring the enhancing action for BMP production of the substance obtained in the step (a) using the measurement method of the present invention (which may be hereinafter referred to as "the preparation method 1 of the present invention"). The preparation method 1 of the present invention is useful in the preparation of a substance for enhancing BMP production in which the enhancing action for BMP production has been tested.

The step (a) can be carried out, for instance, by obtaining a substance for enhancing BMP production in accordance with the above-mentioned screening method of the present invention.

In the step (b), for instance, the component for enhancing BMP production can be isolated and purified from the resulting substance for enhancing BMP production. In other words, the desired component can be obtained by measuring the enhancing action for BMP production of the substance obtained in step (a) using the measurement method of the present invention in the step (b), and performing isolation and purification procedures while confirming the degree of purification of the component for enhancing BMP production using the action as an index. The isolation and purification of the component for enhancing BMP production can be carried out in accordance with a known method as mentioned above as a method which can be applied to a processed product derived from a plant, which is the effective ingredient of the present invention.

In addition, as one embodiment of the preparation method of the present invention, there is provided a method for preparing a substance having an enhancing action for BMP production, characterized in that the method comprises the steps of (a) culturing the cell strain of the present invention with contact of a test substance; and (b) measuring an amount of BMP in a culture medium obtained in the step (a), wherein the test substance is determined to have an enhancing action for BMP production when the amount of BMP is larger than that of a case where the cells are cultured without contact of the test substance or with contact of a control substance having an enhancing action for BMP production, thereby giving the test substance as a substance having an enhancing action for BMP production (which is hereinafter referred to as the preparation method 2 of the present invention).

The preparation method 2 of the present invention can be carried out in the same manner as the above-mentioned screening method of the present invention. In the preparation method 2 of the present invention, when the test substance is determined to have an enhancing action for BMP production, the test substance is obtained as a substance having an enhancing action for BMP production. The resulting substance having an enhancing action for BMP production is further subjected to the preparation method 1 of the present invention, and the isolation and purification of the component for enhancing BMP production may be carried out.

No toxicity is found even when the above-mentioned effective ingredient usable in the present invention is administered to an organism in an amount effective for the exhibition of its action. For instance, in the case of oral administration, no cases of deaths are found even when each of a water extract from *Angelica keiskei koidz.,Apium, Lilium, aloe or Artemisia L.* is administered to a mouse at 1 g/kg in a single dose. In addition, no cases of deaths are found even when the above-mentioned effective ingredient is orally administered to a rat at 1 g/kg in a single dose.

### EXAMPLES

The present invention will be described more concretely hereinbelow by means of the examples, but the present invention is by no means limited to these descriptions. Unless specified otherwise, "%" in these examples means "% by volume."

### Example 1 Preparation of (E)-1-(5, 6, 7, 8, 8a,10a-hexahydro-1, 7-dihydroxy-8, 8, 10a-trimethyl-9H-xanthen-4-yl)-3-(4-hydroxyphenyl)-2-propen-1-one

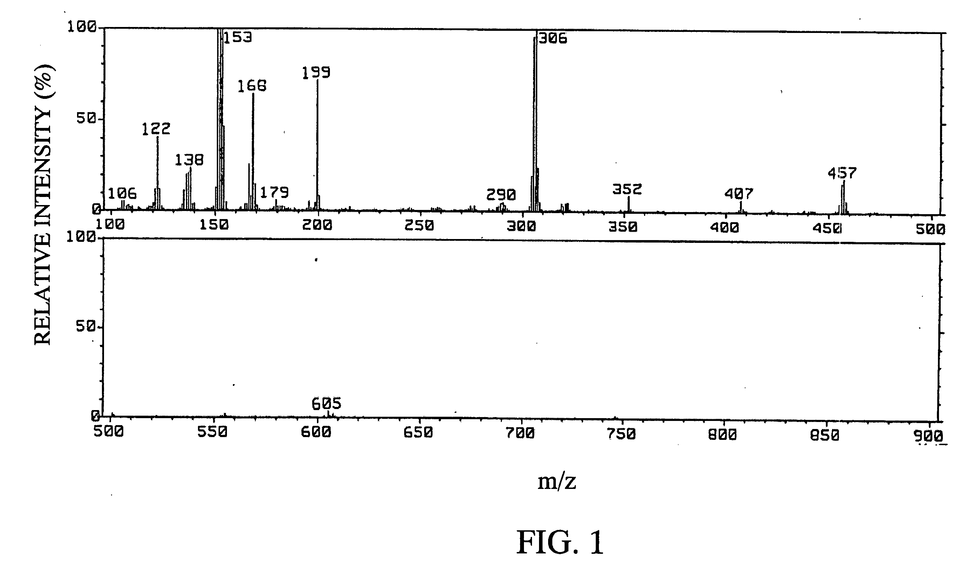

(1) Twenty-four liters of ethyl acetate was added to 5.8 kg of dry powder of root portions of *Angelica keiskei* koidz., and extraction was carried out with stirring at room temperature for 3 hours. After suction filtration, the mixture was separated into an ethyl acetate extract and residue. After the ethyl acetate extract was concentrated under reduced pressure with a rotary evaporator, the concentrate was dissolved in chloroform, and the entire amount of the solution was absorbed to silica gel BW-300SP (manufactured by Fuji Silysia Chemical Ltd.: 750 ml). Next, the absorbed substances were eluted stepwise with hexane : chloroform = 2 : 5 (750 ml), chloroform (1000 ml), chloroform : methanol = 10 : 1 (1100 ml) in that order.
(2) After the fraction eluted with chloroform : methanol = 10:1 obtained in item (1) of Example 1 was concentrated to dryness, the concentrate was dissolved in 30 ml of chloroform. A half the volume of the solution was absorbed to silica gel (BW-300SP, 300 ml). Next, the absorbed substances were eluted stepwise with chloroform (1800 ml), chloroform : methanol = 500 : 7 (300 ml), ethyl acetate (300 ml) in that order.
(3) After the fraction eluted with ethyl acetate obtained in item (2) of Example 1 was concentrated to dryness, the concentrate was dissolved in chloroform : methanol = 50 : 1 (20 ml), and the solution was absorbed to silica gel (BW-300SP, 300 ml). Subsequently, the absorbed substances were eluted stepwise with chloroform : methanol = 500 : 10 (1200 ml),
   chloroform : methanol = 500 : 13 (500 ml), chloroform : methanol = 500: 19 (500 ml), chloroform : methanol = 500 : 22 (800 ml), ethyl acetate (500 ml) in that order, and fractions were collected for every 18 ml portion per fraction.
(4) The silica chromato-fractions Nos. 115 to 155 obtained in item (3) of Example 1 were combined, concentrated under reduced pressure, and dissolved in 7 ml of dimethyl sulfoxide. A half the volume of the solution was fractionated by using reversed phase chromatography. As the resin, Cosmosil 140 C18-OPN (manufactured by nakalai tesque Inc.: amount of resin 50 ml) was used. Subsequently, elution was carried out sequentially with distilled water (40 ml), a 20% aqueous ethanol solution (50 ml), a 30% aqueous ethanol solution (50 ml), a 50% aqueous ethanol solution (first-time elution: 50 ml, and then second-time elution: 50 ml), ethanol (50 ml) in that order.
(5) After 50 ml of the first-time elution portion of the fraction eluted with a 50% aqueous ethanol solution in item (4) of Example 1 was concentrated under reduced pressure, the concentrate was dissolved in 3 ml of a 50% aqueous ethanol solution, and fractionation was carried out by using reversed phase chromatography. The conditions therefor are given below. The column used was TSK gel ODS-80Ts (21.5 mm × 30 cm: manufactured by Tosoh Corporation). A solution prepared by mixing distilled water and acetonitrile in a volume ratio of 1 : 1 was used as a solvent. The elution rate was 5 ml/min., and detection was carried out at 215 nm. Reversed phase chromato-fractions 1 to 5 were collected using ultraviolet absorbance of the eluate as an index.
(6) The mass spectrum (MS) of the reversed phase chromato-fraction 2 (a fraction containing a peak detected at a retention time of 24.1 minutes) obtained in item (5) of Example 1 was measured with a mass spectrometer (DX302: manufactured by JEOL LTD.) by FAB-MS technique. As the matrix, m-nitrobenzyl alcohol was used. As a result, a peak of m/z 407(M-H)⁻ was detected. Figure 1 shows the mass spectrum. In Figure 1, the axis of abscissas is m/z value, and the axis of ordinates is relative intensity. Next, the structure of the reversed phase chromato-fraction 2 was analyzed by measuring various kinds of NMR spectrum with a nuclear magnetic resonance (NMR) spectrometer (Model AVANCE 600: manufactured by Bruker BIOSPIN). The signals of NMR are shown below. In addition, the numbers of the peaks are shown in the following formula (B).

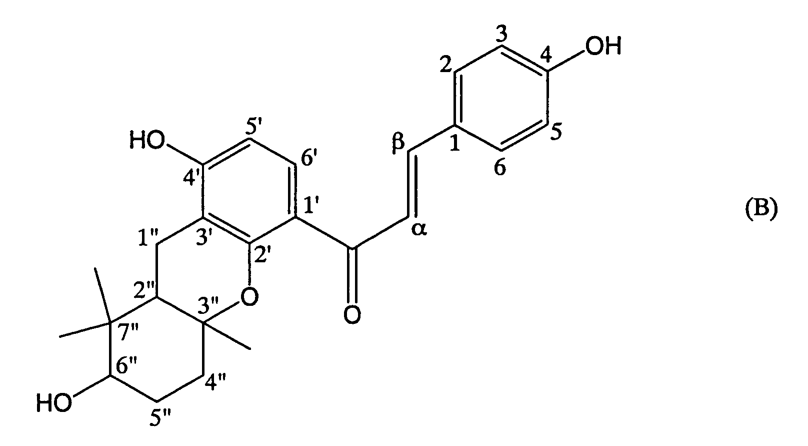

¹H-NMR: δ 0.81(3H,s,7"-CH₃), 1.03(3H,s,7"-CH₃), 1.25(3H,s,3"-CH₃), 1.54(1H,m,5"-H), 1.61(1H,dd,J=4.8,13.2 Hz,2"-H), 1.71(1H,m,5"-H), 1.75(1H,m,4"-H), 1.87(1H,m,4"-H), 2.34(1H,dd,J=13.2,16.8 Hz,1"-H), 2.67(1H,dd,J=4.8,16.8 Hz,1"-H), 3.27(1H,m,6"-H), 4.65(1H,d,J=4.8 Hz,6"-OH), 6.47(1H,d,J=8.4 Hz,5'-H), 6.83(2H,d,J=8.4 Hz,3-H and 5-H), 7.39(1H,d,J=8.4 Hz,6'-H), 7.42(1H,d,J=15.6 Hz,β-H), 7.48(1H,d,J=15.6 Hz,α-H), 7.51(2H,d,J=8.4 Hz,2-H and 6-H), 9.97(1H,br-s,4-OH), 10.22(1H,br-s,4'-OH)

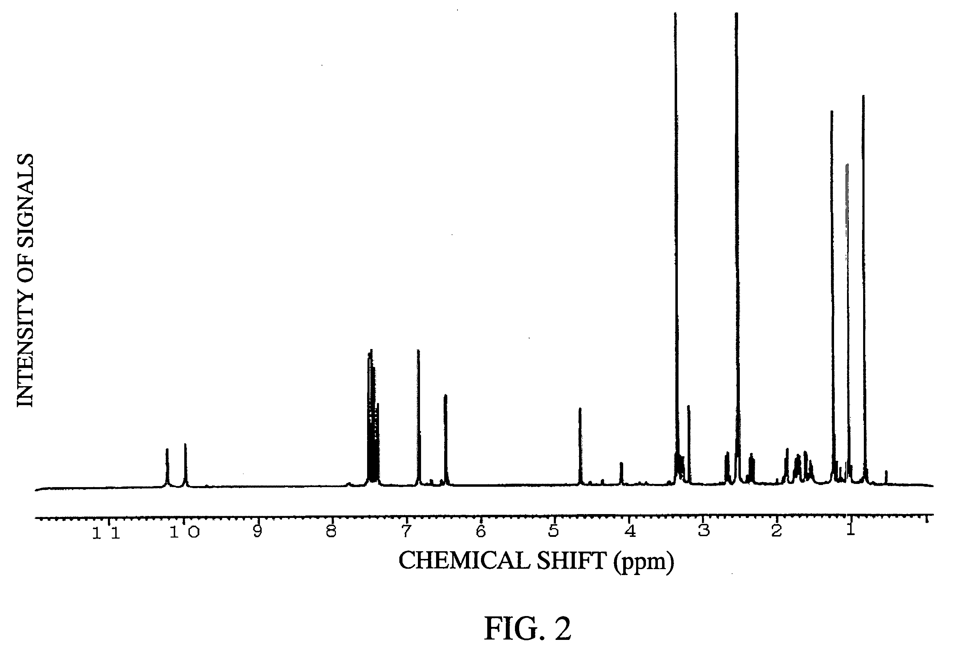

Here, in ¹H-NMR, the sample was dissolved in deuterated dimethyl sulfoxide, and the chemical shift of the residual proton in deuterated dimethyl sulfoxide was expressed as 2.51 ppm. Figure 2 shows ¹H-NMR spectrum. In Figure 2, the axis of abscissas is chemical shift, and the axis of ordinates is intensity of signal.

¹³C-NMR: δ 15.3(7"-CH₃), 18.8(1"-C), 20.7(3"-CH₃), 28.1(7"-CH₃), 28.9(5"-C), 38.3(4"-C), 38.9(7"-C), 46.4(2"-C), 76.8(6"-C), 77.9(3"-C), 107.7(5'-C), 110.4(3'-C), 116.8(3-C and 5-C), 120.8(1'-C), 125.2(α-C), 127.1(1-C), 130.2(6'-C), 130.8(2-C and 6-C), 141.2(β-C), 154.9(2'-C), 160.3(4-C), 160.6(4'-C), 189.8(C=O)

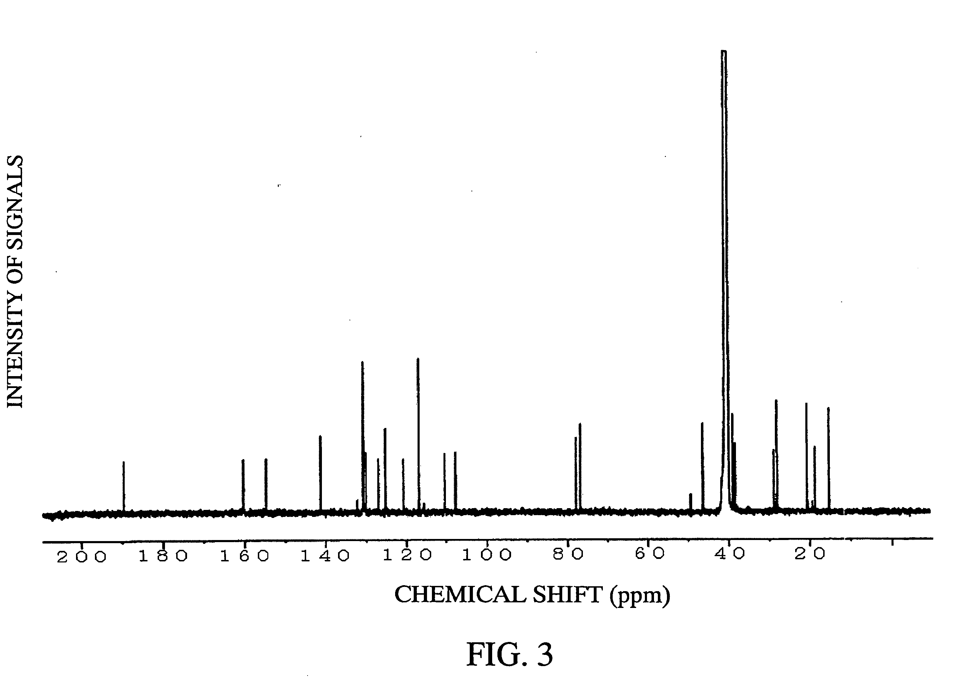

Here, in ¹³C-NMR, the sample was dissolved in deuterated dimethyl sulfoxide, and the chemical shift of deuterated dimethyl sulfoxide was expressed as 40.2 ppm. Figure 3 shows ¹³C-NMR spectrum. In Figure 3, the axis of abscissas is chemical shift, and the axis of ordinates is intensity of signal.

From the above results of the mass spectrum analysis and NMR spectrum analysis carried out for the reversed phase chromato-fraction 2, it was confirmed that the reversed phase chromato-fraction 2 is (E)-1-(5, 6, 7, 8, 8a, 10a-hexahydro-1,7-dihydroxy-8, 8, 10a-trimethyl-9H-xanthen-4-yl)-3-(4-hydroxyphenyl)-2-propen-1-one (molecular weight: 408, hereinafter referred to as compound **a**).

### Example 2 Actions for Induction of Differentiation of ST-2 cells into Osteoblasts by Compound a

(1) Mouse interstitial cell strain ST-2 was suspended in DMEM medium (manufactured by Bio Whittaker) containing 10% fetal bovine serum (manufactured by Bio Whittaker) so as to have a concentration of 3 × 10⁴ cells/ml. The suspension was put to a 96-well plate in an amount of 0.1 ml per well, and the cells were cultured sterilely. After the cells were cultured for 2 days, the medium was exchanged with a fresh medium. The compound a derived from root portions of *Angelica keiskei* koidz. obtained in Example 1 was added thereto as a sample, and the cells were cultured for 3 days. Subsequently, differentiation of ST-2 cells into osteoblasts was measured using alkaline phosphatase as an index. The cells were washed once with PBS, 100 µl of a reaction substrate solution (100 mM diethanolamine buffer, pH 10.0, 2 mM magnesium chloride, 1 mM p-nitrophenyl phosphate) was added thereto, and the reaction was carried out at 37°C for 30 minutes. Next, the reaction was terminated with adding 100 µl of 0.2 N sodium hydroxide, and the amount of p-nitrophenol which was released was determined by determining absorbance at 405 nm. The activity for induction of differentiation into osteoblasts was shown by setting a group without addition of the sample as a control, and defining alkaline phosphatase activity of the control as 100%. The amount of the sample was as shown in Table 1. The experiments were carried out twice, and the average was taken. As a result, it was revealed that the compound a induces differentiation into osteoblasts in a concentration-dependent manner. The results are shown in Table 1.

**Table 1**

| Activities for Induction of Differentiation of ST-2 Cells into Osteoblasts by Compound **a** | |
|---|---|
| Amount (µM) | Alkaline Phosphatase Activity (%) |
| 0 | 100 |
| 0.313 | 219.4 |
| 0.625 | 259.6 |
| 1.25 | 358.3 |

### Example 3 Actions for Induction of Differentiation of MC3T3-E1 Cells into Osteoblasts by Compound a

Mouse pre-osteoblast strain MC3T3-E1 was suspended in DMEM medium containing 10% fetal bovine serum so as to have a concentration of 3 × 10⁴ cells/ml. The suspension was put to a 96-well plate in an amount of 0.1 ml per well, and the cells were cultured sterilely. After the cells were cultured for 2 days, the medium was exchanged with a fresh medium. The compound a derived from root portions of *Angelica keiskei* koidz. obtained in Example 1 was added thereto as a sample, and the cells were cultured for 5 days. Subsequently, the differentiation into osteoblasts was determined using alkaline phosphatase as an index in the same manner as in Example 2. As a result, it was revealed that the compound a induces the differentiation into osteoblasts in a concentration-dependent manner. The results are shown in Table 2.

**Table 2**

| Activities for Induction of Differentiation of MC3T3-E1 Cells into Osteoblasts by Compound **a** | |
|---|---|
| Amount (µM) | Alkaline Phosphatase Activity (%) |
| 0 | 100 |
| 1.25 | 171.0 |
| 2.5 | 292.2 |

### Example 4 Enhancing Actions for BMP-2 Production of Angelica keiskei Koidz.

(1) Forty milliliters of water was added to 2 g of pulverized dry substance of root portions of *Angelica keiskei* koidz., and extraction was carried out with stirring at 60°C for 30 minutes. Next, the centrifugation was carried out to separate the extract into supernatant and precipitates. The same extraction procedure was repeated for the precipitates twice. The resulting supernatant was collected and concentrated to a volume of 10 ml, to give a water extract from root portions of *Angelica keiskei* koidz.
(2) Human osteosarcoma cell strain HuO9 was suspended in DMEM medium containing 10% fetal bovine serum so as to have a concentration of 1 × 10⁵ cells/ml. The suspension was put to a 96-well plate in an amount of 0.1 ml per well, and the cells were cultured sterilely. After the cells were cultured for 2 days, the medium was exchanged with a fresh medium. The water extract from root portions of *Angelica keiskei* koidz. obtained in item (1) of Example 4 was added thereto as a sample, and the cells were cultured for 48 hours. Next, the concentration of bone morphogenetic protein-2 (BMP-2) in the culture medium was measured by enzyme immunoassay method (BMP-2 Immunoassay: manufactured by GT). The enhancing activity for BMP-2 production was shown by setting a group without addition of the sample as a control, and defining the BMP-2 concentration in the cell culture medium (the amount of BMP-2 production of cells) as 100%. The amount of the sample was as shown in Table 3. The experiments were carried out twice, and the average was taken. As a result, it was revealed that the water extract from root portions of *Angelica keiskei* koidz. obtained in item (1) of Example 4 enhances BMP-2 production in a concentration-dependent manner. The results are shown in Table 3.

**Table 3**

| Enhancing Actions for BMP-2 Production by the Water Extract from Root Portions of *Angelica keiskei* Koidz. | |
|---|---|
| Amount (%) | Enhancing Activity for BMP-2 Production (%) |
| 0 | 100.0 |
| 0.625 | 192.8 |
| 1.25 | 277.7 |
| 2.5 | 405.0 |
| The amount of BMP-2 production in the control was 0.270 ng/ml. | |

### Example 5 Actions for Induction of Differentiation into Osteoblasts by Angelica keiskei Koidz.

Mouse embryonal cell strain C3H10T1/2 was suspended in DMEM medium containing 10% fetal bovine serum so as to have a concentration of 3 × 10⁴ cells/ml. The suspension was put to a 96-well plate in an amount of 0.1 ml per well, and the cells were cultured sterilely. After the cells were cultured for 3 days, the medium was exchanged with a fresh medium. The water extract from root portions of *Angelica keiskei* koidz. obtained in item (1) of Example 4 was added thereto as a sample, and the cells were cultured for 6 days. Subsequently, differentiation into osteoblasts was measured using alkaline phosphatase as an index in the same manner as in Example 2. As a result, it was revealed that the water extract from root portions of *Angelica keiskei* koidz. obtained in item (1) of Example 4 induces differentiation into osteoblasts in a concentration-dependent manner. The results are shown in Table 4.

**Table 4**

| Actions for Induction of Differentiation into Osteoblasts by the Water Extract from Root Portions of *Angelica keiskei* Koidz. | |
|---|---|
| Amount (%) | Alkaline Phosphatase Activity (%) |
| 0 | 100.0 |
| 0.025 | 137.1 |
| 0.05 | 153.4 |
| 0.1 | 159.4 |
| 0.2 | 202.9 |

### Example 6 Enhancing Actions for BMP-2 Production by Cosmosil-Fractionated Product Derived from Water Extract from Root Portions of Angelica keiskei Koidz.

(1) Thirty liters of water was added to 3 kg of pulverized dry substance of root portions of *Angelica keiskei* koidz., and extraction was carried out with stirring at 60°C for 60 minutes. Next, the centrifugation was carried out to separate the extract into supernatant and precipitates. Twenty liters of water was added to the precipitates, and extraction was carried out with stirring at room temperature for 60 minutes. Next, the centrifugation was carried out to separate the extract into supernatant and precipitates. The supernatant was combined, to give 45 *l* of a water extract from root portions of *Angelica keiskei* koidz.
(2) The water extract from root portions of *Angelica keiskei* koidz. obtained in item (1) of Example 6 was fractionated by using reversed phase chromatography. The conditions therefor are given below. As the resin, Cosmosil 140 C18-OPN (amount of resin: 700 ml) was used. The amount 1.51 *l* of the water extract from root portions of *Angelica keiskei* koidz. was eluted with water (2.10 *l*), 10% ethanol (2 *l*), 20% ethanol (2.3 *l*), 40% ethanol (2 *l*), 60% ethanol (3.5 *l*) as developing solvents in that order, to give each of the Cosmosil-fractionated products.
(s) The amount 1/50 of each the Cosmosil-fractionated products obtained in item (2) of Example 6 was concentrated to a volume of 10 ml. The enhancing activity for BMP-2 production of each concentrate was measured in the same manner as in item (2) of Example 4. As a result, it was revealed that a fraction eluted with water, a fraction eluted with 10% ethanol, and a fraction eluted with 20% ethanol have enhancing activities for BMP-2 production. The results are shown in Table 5.

**Table 5**

| Fraction | Sample Concentration (%) | Enhancing Activity for BMP-2 Production (%) |
|---|---|---|
| | 0 | 100 |
| Fraction Eluted with Water | 1.25 | 438.5 |
| | 2.5 | 753.7 |
| | 5 | 1079 |
| | 10 | 1064 |
| Fraction Eluted with 10% | 1.25 | 198.2 |
| Ethanol | 2.5 | 283.5 |
| | 5 | 394.6 |
| | 10 | 637.5 |
| Fraction Eluted with 20% | 1.25 | 180.1 |
| Ethanol | 2.5 | 167.2 |
| | 5 | 244.7 |
| | 10 | 367.5 |
| The amount of BMP-2 production of the control was 0.126 ng/ml. | | |

### Example 7 Enhancing Actions for BMP-2 Production by Silica Column-Fractionated Product Derived from Water Extract from Root Portions of Angelica keiskei Koidz.

(1) Forty-one liters of the water extract from root portions of *Angelica keiskei* koidz. obtained in item (1) of Example 6 was concentrated to a volume of 191, to give a concentrate of the water extract from root portions of *Angelica keiskei* koidz.
(2) Two-hundred milliliters of ethanol was added to 300 ml of the concentrate of the water extract from root portions of *Angelica keiskei* koidz. obtained in item (1) of Example 7. After the mixture was allowed to stand at room temperature for 1 hour, the centrifugation was carried out to separate the mixture into supernatant and precipitates. The same procedure was repeated for a mixture prepared by adding 300 ml of distilled water to the precipitates. The resulting supernatant was combined, and concentrated to dryness. Thereafter, the concentrate was dissolved in 108 ml of distilled water, to give an ethanol precipitate-processed product derived from the water extract from root portions of *Angelica keiskei* koidz.
(3) The ethanol precipitate-processed product derived from the water extract from root portions of *Angelica keiskei* koidz. obtained in item (2) of Example 7 was fractionated by using silica chromatography. The conditions therefor are given below. After 25 ml of the ethanol-precipitate processed product derived from the water extract from root portions of *Angelica keiskei* koidz. was concentrated to dryness, the concentrate was dissolved in a solution prepared by mixing chloroform and ethanol in a volume ratio of 6 : 3, and absorbed to silica gel BW-300SP (amount of resin: 300 ml). Next, the absorbed substances were eluted stepwise with chloroform : ethanol = 6 : 3 (600 ml), chloroform : ethanol : water = 30 : 15 : 1 (900 ml), chloroform : ethanol : water = 12 : 8 : 1 (500 ml), ethanol: water = 10 : 1 (600 ml), ethanol: water = 20 : 3 (200 ml), ethanol : water = 4 : 1 (250 ml), ethanol : water = 1 : 1 (300 ml) in that order.
   The latter half of the eluted portion (200 ml) which was eluted with chloroform : ethanol : water = 12 : 8 : 1, and the first half of the eluted portion (200 ml) which was eluted with ethanol: water =10 : 1, were combined, to give a silica column-eluted fraction 1. The latter half of the eluted portion (400 ml) which was eluted with ethanol: water = 10 : 1, the eluted portion which was eluted with ethanol : water = 20 : 3, and the eluted portion which was eluted with ethanol : water =4:1 were combined, to give a silica column-eluted fraction 2. The eluted portion which was eluted with ethanol: water = 1 : 1 was obtained as a silica column-eluted fraction 3.
(4) The amount 1/5 of each of the silica column fractionated-products obtained in item (3) of Example 7 was concentrated to a volume of 1.25 ml. The enhancing activity for BMP-2 production of each concentrate was measured in the same manner as in item (2) of Example 4. As a result, it was revealed that the fractions shown in Table 6 have enhancing activities for BMP-2 production.

**Table 6**

| Fraction (Composition of Elution Solvent) | Sample Concentration (%) | Enhancing Activity for BMP-2 Production (%) |
|---|---|---|
| | 0 | 100 |
| Fraction 1 | 0.625 | 152.2 |
| (Chloroform : Ethanol : Water = 12 : 8 : 1, | 1.25 | 185.1 |
| Ethanol : Water = 10 : 1) | 2.5 | 307.5 |
| Fraction 2 | 0.625 | 191.0 |
| (Ethanol : Water = 10 : 1, 20 : 3, 4 : 1) | 1.25 | 310.4 |
| | 2.5 | 492.5 |
| | 5 | 558.2 |
| Fraction 3 | 0.625 | 244.8 |
| (Ethanol: Water = 1 : 1) | 1.25 | 420.9 |
| | 2.5 | 671.6 |
| | 5 | 803.0 |
| The amount of BMP-2 production of the control was 0.096 ng/ml. | | |

### Example 8 Enhancing Actions for BMP-2 Production of Water Extract from Leaf Portions of Apium

(1) Forty milliliters of water was added to 2 g of pulverized dry substance of leaf portions of *Apium,* and extraction was carried out with stirring at 60°C for 30 minutes. The centrifugation was carried out to separate the extract into supernatant and precipitates. The same extraction procedure was repeated twice for the precipitates. The resulting supernatant was combined and concentrated to a volume of 10 ml, to prepare a water extract from leaf portions *of Apium.*
(2) The enhancing activity for BMP-2 production of the water extract from leaf portions of *Apium* prepared in item (1) of Example 8 was measured in the same manner as in item (2) of Example 4. As a result, it was revealed that the water extract from leaf portions of *Apium* has enhancing activity for BMP-2 production.

**Table 7**

| Enhancing Actions for the Water Extract from Leaf Portions of *Apium* for BMP-2 Production | |
|---|---|
| Amount (%) | Enhancing Activity for BMP-2 Production (%) |
| 0 | 100 |
| 2.5 | 171.8 |
| 5.0 | 527.0 |
| The amount of BMP-2 production of the control was 0.119 ng/ml. | |

### Example 9 Enhancing Actions for BMP-2 Production of Water Extract from Aloe

(1) Forty milliliters of water was added to 2 g of pulverized dry substance of aloe, and extraction was carried out with stirring at 60°C for 30 minutes. Thereafter, the centrifugation was carried out to separate the extract into supernatant and precipitates. The same extraction procedure was repeated twice for the precipitates. The resulting supernatant was combined and concentrated to a volume of 10 ml, to give a water extract from aloe.
(2) The enhancing activity for BMP-2 production of the water extract from aloe prepared in item (1) of Example 9 was measured in the same manner as in item (2) of Example 4. As a result, it was revealed that the water extract from aloe has enhancing activity for BMP-2 production.

**Table 8**

| Enhancing Actions for BMP-2 Production of the Water Extract from Aloe | |
|---|---|
| Amount (%) | Enhancing Activity for BMP-2 Production (%) |
| 0 | 100 |
| 5.0 | 238.5 |
| The amount of BMP-2 production of the control was 0.274 ng/ml. | |

### Example 10 Enhancing Actions for BMP-2 Production of Water Extract from Artemisia L.

(1) Forty milliliters of water was added to 2 g of pulverized dry substance of *Artemisia L.,* and extraction was carried out with stirring at 60°C for 30 minutes. Thereafter, the centrifugation was carried out to separate the extract into supernatant and precipitates. The same extraction procedure was repeated twice for the precipitates. The resulting supernatant was combined and concentrated to a volume of 10 ml, to give a water extract from *Artemisia L.*
(2) The enhancing activity for BMP-2 production of the water extract from *Artemisia L.* prepared in item (1) of Example 10 was measured in the same manner as in item (2) of Example 4. As a result, it was revealed that the water extract from *Artemisia L.* has enhancing activity for BMP-2 production.

**Table 9**

| Enhancing Actions for BMP-2 Production of the Water Extract from *Artemisia L.* | |
|---|---|
| Amount (%) | Enhancing Activity for BMP-2 Production (%) |
| 0 | 100 |
| 0.25 | 383.4 |
| 0.5 | 461.5 |
| The amount of BMP-2 production of the control was 0.119 ng/ml. | |

### Example 11 Actions of Induction of Differentiation into Osteoblasts by Water Extract from Aloe

The actions of induction of differentiation into osteoblasts by the water extract from aloe prepared in item (1) of Example 9 were measured in the same manner as in Example 5. As a result, it was revealed that the water extract from aloe obtained in item (1) of Example 9 induces differentiation into osteoblasts in a concentration-dependent manner. The results are shown in Table 10.

**Table 10**

| Actions of Induction of Differentiation into Osteoblasts by the Water Extract from Aloe | |
|---|---|
| Amount (%) | Alkaline Phosphatase Activity (%) |
| 0 | 100 |
| 0.125 | 118.3 |
| 0.25 | 125.4 |
| 0.5 | 131.7 |
| 1 | 184.6 |
| 2 | 249.6 |

### Example 12 Actions of Induction of Differentiation into Osteoblasts by Water Extract from Flowers of Lilium

(1) Forty milliliters of water was added to 2 g of pulverized dry substance of flower moiety of *Lilium,* and extraction was carried out with stirring at 60°C for 30 minutes. Thereafter, the centrifugation was carried out to separate the extract into supernatant and precipitates. The same extraction procedure was repeated twice for the precipitates. The resulting supernatant was combined and concentrated to a volume of 10 ml, to give a water extract from flowers of *Lilium.*
(2) The actions of induction of differentiation into osteoblasts by the water extract from flower of *Lilium* prepared in item (1) of Example 12 were measured in the same manner as in Example 5. As a result, it was revealed that the water extract from flowers of *Lilium* obtained in item (1) of Example 12 induces differentiation into osteoblasts in a concentration-dependent manner. The results are shown in Table 11.

**Table 11**

| Actions of Induction of Differentiation into Osteoblasts by the Water Extract from Flowers of *Lilium* | |
|---|---|
| Amount (%) | Alkaline Phosphatase Activity (%) |
| 0 | 100 |
| 0.013 | 97.9 |
| 0.025 | 137.5 |
| 0.05 | 175.7 |
| 0.1 | 280.8 |
| 0.2 | 290.4 |

### Example 13 Synergistic Effects of Actions of Induction of Differentiation into Osteoblasts by Water Extract from Root Portions of Angelica keiskei Koidz. and Recombinant Human BMP-2

Mouse embryonal cell strain C3H10T1/2 was suspended in DMEM medium containing 10% fetal bovine serum so as to have a concentration of 3 × 10⁴ cells/ml. The suspension was put to a 96-well plate in an amount of 0.1 ml per well, and the cells were cultured sterilely. After the cells were cultured for 4 days, the medium was exchanged with a fresh medium. The water extract from root portions of *Angelica keiskei* koidz. obtained in item (1) of Example 4 and recombinant human BMP-2 (manufactured by GT) were added thereto as a sample, and the cells were cultured for 11 days. Subsequently, differentiation into osteoblasts was measured using alkaline phosphatase activity as an index in the same manner as in Example 5. As a result, it was revealed that the recombinant human BMP-2 induces differentiation into osteoblasts in a concentration-dependent manner, and when it is used together with the water extract from root portions of *Angelica keiskei* koidz., the action of induction of differentiation into osteoblasts is enhanced. The results are shown in Table 12.

**Table 12**

| Actions of Induction of Differentiation into Osteoblasts by Water Extract from Root Portions of *Angelica keiskei* Koidz. and Human BMP-2 | | |
|---|---|---|
| Recombinant Human BMP-2 (ng/ml) | Water Extract from Root Portions of *Angelica keiskei* Koidz. (%) | Alkaline Phosphatase Activity (%) |
| 0 | 0 | 100.0 |
| 0 | 0.05 | 190.5 |
| 500 | 0 | 379.6 |
| 500 | 0.05 | 745.6 |
| 1000 | 0 | 687.1 |
| 1000 | 0.05 | 1404.1 |

### Example 14 Actions for Induction of Differentiation of Mesenchymal Stem Cells into Osteoblasts by Water Extract from Root Portions of Angelica keiskei Koidz.

Human mesenchymal stem cells (manufactured by TAKARA BIO Inc.) was suspended in DMEM medium (manufactured by TAKARA BIO Inc.) containing 10% fetal bovine serum so as to have a concentration of 1.2 × 10⁴ cells/ml. The suspension was put to a 48-well plate in an amount of 0.5 ml per well, and the cells were cultured sterilely. After the cells were cultured for 5 days, the medium was exchanged with a fresh medium containing 5 mM β-glycerophosphoric acid and 100 nM dexamethasone. The water extract from root portions of *Angelica keiskei* koidz. obtained in item (1) of Example 4 was added thereto as a sample, and the cells were cultured. The medium was exchanged every 3 or 4 days. The differentiation of human mesenchymal stem cells into osteoblasts was measured using alkaline phosphatase activity and accumulation of calcium in cells as indexes. The alkaline phosphatase activity was measured in the same manner as in Example 2, and expressed as the amount of p-nitrophenol which was released per minute per well. The cells were washed with PBS, and 6 N hydrochloric acid was added thereto. The mixture was ultrasonically disrupted. The amount of calcium accumulated in cells was measured for the resulting solution using a calcium quantifying kit (Calcium E-test Wako; manufactured by Wako Pure Chemical Industries, Ltd.).

As a result, the water extract from root portions of *Angelica keiskei* koidz. strongly promoted the alkaline phosphatase activity accompanying the differentiation of human mesenchymal stem cells into osteoblasts and the accumulation of calcium accompanying the maturation of osteoblasts over time. The alkaline phosphatase activities are shown in Table 13, and the results of the measurements of accumulation of calcium in cells are shown in Table 14.

**Table 13**

| Water Extract from Root Portions of *Angelica keiskei* Koidz. (%) | Alkaline Phosphatase Activity of Cells (pmol p-nitrophenol/min./well) | | | |
|---|---|---|---|---|
| | Culture Time (day) | | | |
| | 0 | 7 | 14 | 21 |
| 0 | 50.5 | 786.5 | 2490.2 | 3656.0 |
| 0.1 | 50.5 | 2277.7 | 5022.9 | 4724.1 |

**Table 14**

| Water Extract from Root Portions of *Angelica keiskei* Koidz. (%) | Accumulation of Calcium in Cells (µg calcium/well) | | | |
|---|---|---|---|---|
| | Culture Time (day) | | | |
| | 0 | 14 | 21 | 28 |
| 0 | 0.910 | 2.140 | 2.288 | 3.192 |
| 0.1 | 0.910 | 2.575 | 4.832 | 58.127 |

### INDUSTRIAL APPLICABILITY

According to the present invention, there is provided a medicament for treatment or prevention of a disease requiring promotion of osteogenesis or enhancement of BMP production, an agent for promotion of osteogenesis, an agent for enhancement of BMP production, or a food, beverage, or feed, each comprising a processed product derived from a plant belonging to Umbelliferae, Liliaceae, or Compositae. The medicament is useful as a therapeutic agent or prophylactic agent for a disease associated with bone such as osteoporosis or bone fracture. In addition, the agent for promotion of osteogenesis and the agent for enhancement of BMP production can be used as implant or dentifrice used in bone fracture treatment or dental treatment. The agents are also useful for function studies of bone, and screening of an agent for enhancement of BMP production. Also, the food or beverage will be able to, for example, ameliorate symptoms of a disease requiring promotion of osteogenesis or enhancement of BMP production by taking the food or beverage as foodstuff for daily use. The same effects can be expected for the feed by its intake by the animals.

## Claims

1. A therapeutic agent or prophylactic agent for a disease requiring promotion of osteogenesis or enhancement of bone morphogenetic protein production, **characterized in that** the therapeutic agent or prophylactic agent comprises as an effective ingredient a processed product derived from a plant selected from the following (a) to (c):
(a) a processed product derived from a plant belonging to Umbelliferae;
(b) a processed product derived from a plant belonging to Liliaceae; and
(c) a processed product derived from a plant belonging to Compositae.

2. The therapeutic agent or prophylactic agent according to claim 1, wherein the plant is *Angelica keiskei* koidz., *Apium, Lilium,* aloe or *Artemisia L.*

3. An agent for promotion of osteogenesis or an agent for enhancement of bone morphogenetic protein production, **characterized in that** the agent comprises as an effective ingredient a processed product derived from a plant selected from the following (a) to (c):
(a) a processed product derived from a plant belonging to Umbelliferae;
(b) a processed product derived from a plant belonging to Liliaceae; and
(c) a processed product derived from a plant belonging to Compositae.

4. The agent for promotion of osteogenesis or the agent for enhancement of bone morphogenetic protein production according to claim 3, wherein the plant is *Angelica keiskei* koidz., *Apium, Lilium,* aloe or *Artemisia L.*

5. A food, beverage or feed for promotion of osteogenesis or enhancement of bone morphogenetic protein production, **characterized in that** the food, beverage or feed comprises a processed product derived from a plant selected from the following (a) to (c):
(a) a processed product derived from a plant belonging to Umbelliferae;
(b) a processed product derived from a plant belonging to Liliaceae; and
(c) a processed product derived from a plant belonging to Compositae.

6. The food, beverage or feed according to claim 5, wherein the plant is *Angelica keiskei* koidz., *Apium, Lilium,* aloe or *Artemisia L.*

7. A therapeutic agent or prophylactic agent for a disease requiring promotion of osteogenesis or enhancement of bone morphogenetic protein production, **characterized in that** the therapeutic agent or prophylactic agent comprises as an effective ingredient a compound represented by the following formula (A): 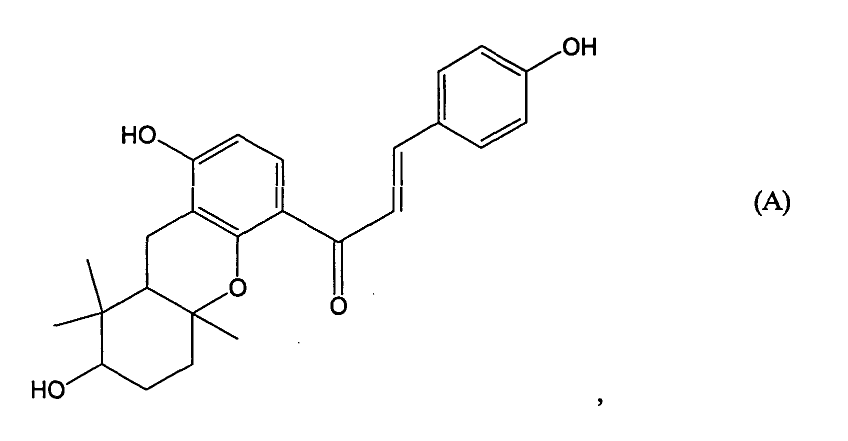 a derivative thereof or a salt thereof.

8. An agent for promotion of osteogenesis or an agent for enhancement of bone morphogenetic protein production, **characterized in that** the agent comprises as an effective ingredient the compound represented by the formula (A) as defined in claim 7, a derivative thereof or a salt thereof.

9. A food, beverage or feed for promotion of osteogenesis or enhancement of bone morphogenetic protein production, **characterized in that** the food, beverage or feed comprises the compound represented by the formula (A) as defined in claim 7, a derivative thereof or a salt thereof.

10. A method for measuring an enhancing action for bone morphogenetic protein production, **characterized in that** the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a) as an index for an enhancing action for BMP production of the test substance.

11. A method for screening a substance having an enhancing action for bone morphogenetic protein production, **characterized in that** the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a) wherein the test substance is determined to have an enhancing action for BMP production when the amount of BMP is larger than that of a case where the cells are cultured without contact of the test substance or with contact of a control substance having an enhancing action for BMP production.

12. A method for preparing a substance having an enhancing action for bone morphogenetic protein production, **characterized in that** the method comprises the following steps of:
(a) obtaining a substance having an enhancing action for bone morphogenetic protein production; and
(b) measuring the enhancing action for bone morphogenetic protein production of the substance obtained in the step (a) using the measurement method as defined in claim 10.

13. A method for preparing a substance having an enhancing action for bone morphogenetic protein production, **characterized in that** the method comprises the following steps of:
(a) culturing hybridoma obtained by using HuO9 cells or a substrain thereof, or any one of cell strains therefrom with contact of a test substance; and
(b) measuring an amount of BMP in a culture medium obtained in the step (a), wherein the test substance is determined to have an enhancing action for BMP production when the amount of BMP is larger than that of a case where the cells are cultured without contact of the test substance or with contact of a control substance having an enhancing action for BMP production, thereby giving the test substance as a substance having an enhancing action for BMP production.
